# EUROPEAN PATENT SPECIFICATION

(11) **EP 2 488 134 B1**
(45) Date of publication and mention of the grant of the patent: **07.08.2013**
(21) Application number: 10772970.9
(22) Date of filing: 13.10.2010
(51) Int. Cl.: A61F 9/007, A61B 3/16

(54) **OPHTHALMOSURGICAL MEASURING DEVICE**
AUGENCHIRURGISCHE MESSVORRICHTUNG
DISPOSITIF DE MESURE OPHTALMOCHIRURGICALE

(30) Priority: 14.10.2009 DE 102009049430; 14.10.2009 US 251391 P
(43) Date of publication of application: 22.08.2012
(73) Proprietor: Carl Zeiss Meditec AG, 07745 Jena (DE)
(72) Inventor: KUEBLER, Christoph, 73447 Oberkochen (DE); EICHLER, Michael, 73434 Aalen (DE); MAIER, Tobias, 70178 Stuttgart (DE)
(74) Representative: Carl Zeiss AG - Patentabteilung
(86) International application number: PCT/EP2010/006247
(87) International publication number: WO 2011/045033

(56) References cited:
- US-A- 4 870 964
- US-A- 5 700 240
- US-A- 5 904 669
- US-A1- 2007 010 730

## Description

The invention relates to an ophthalmosurgical device, an ophthalmosurgical system having such a device, and a method for operating such a device.

There are several ophthalmosurgical techniques for treating a clouded lens of the human eye. The most widely used technique is phacoemulsification, in which a thin tip is introduced into the diseased lens and is excited with ultrasound vibrations. In its immediate environment, the vibrating tip emulsifies the lens in such a way that the resulting lens fragments can be sucked through a line by a pump. When the lens has been completely emulsified, a new and artificial lens can be inserted into the empty capsular bag, such that a patient treated in this way can recover good visual acuity.

In phacoemulsification, a device is used that generally has a vibratable tip in a handpiece, a flushing line (irrigation line) for conveying irrigation fluid to the lens to be treated, and a suction line (aspiration line) for transporting emulsified lens fragments into a collecting vessel. During transport into the collecting vessel, it can happen that a lens fragment blocks the inlet area of the handpiece tip. With a suction pump running continuously, an underpressure therefore builds up downstream in the aspiration line. The lens fragment can be broken into smaller segments, for example by continued ultrasound vibrations of the tip, as a result of which the blockage (occlusion) is ended abruptly. The underpressure that has built up in the aspiration line has the effect that, when such an occlusion has been broken through, a relatively large amount of fluid is sucked out of the eye in a very short time. This may result in a collapse of the anterior chamber of the eye. It is then possible that the capsular bag will be drawn toward the tip of the handpiece and be punctured by the tip. With such damage to the capsular bag, it is also possible for a tip that has penetrated too deeply to cause damage to the vitreous body lying behind the capsular bag.

It is therefore important to avoid a collapse of the anterior chamber of the eye when an occlusion is broken through. A precondition for this is that the break-through of the occlusion is identified quickly. One possibility is to precisely detect the pressure profile in the aspiration line. If the underpressure quickly decreases, this is an indication that an occlusion has been broken through. Such information can be used to change the vibrations of the tip of the handpiece or to change the volumetric flow in the irrigation line or aspiration line. In the prior art, this is described, for example, in US 5,700,240.

A disadvantage of measuring the pressure in the aspiration line is that the onset of the occlusion and the end of an occlusion are only detected relatively late. If a blockage of the needle occurs, a relatively high underpressure in the aspiration line builds up only slowly, depending on the efficiency of the suction pump. Although the high underpressure in the aspiration line decreases relatively quickly when an occlusion is broken through, a much quicker change in pressure takes place in the irrigation line, with the result that valuable time is lost before the break-through of an occlusion can be reliably detected. In this "dead time", there is a great danger of the above-described problems occurring, namely damage to the capsular bag or to the vitreous body lying behind the latter. A sensor for pressure measurement could now likewise be placed in the irrigation line. However, a disadvantage of such a solution is that, on the one hand, two pressure sensors would have to be used, which would result in a very expensive design, and, on the other hand, the signals from two pressure sensors with unavoidably different time constants would have to be processed, and this would result in a relatively high outlay in terms of control elements.

It is an object of the invention to make available an ophthalmosurgical device with which the onset and break-through of an occlusion can be detected very quickly, very precisely, inexpensively and with minimal outlay in terms of control elements. It is also an object to make available an ophthalmosurgical system having such a device, and a method for operating such a device.

These objects are achieved, in terms of the device, the system and the method, by the subject matter of the independent claim. Advantageous developments are set forth in the dependent claims. The two part form of claim 1 is based on US 4810964.

The ophthalmosurgical device according to the invention has : an irrigation line through which irrigation fluid can be transported, an aspiration line through which aspiration fluid can be transported to a suction pump, and a sensor with which a differential pressure between the irrigation line and aspiration line can be detected the being directly connected to the irrigation line and the aspiration line. In such a device, the pressure in the aspiration line or the pressure in the irrigation line is not detected directly. Instead of using two sensors, which always have different time constants and whose signals are therefore difficult to process together, only one sensor is used according to the invention. With this sensor, it is not possible to detect the relative pressure in the aspiration line or irrigation line, for example in relation to atmospheric pressure. Rather, the two pressures are compared with each other and the difference is formed. A reference point, which for example can be the ambient pressure in the case of a relative pressure sensor, is not available in the measuring device according to the invention with the sensor measuring the differential pressure. The advantages are, on the one hand, that only a single sensor is needed, as a result of which an inexpensive solution is achieved. On the other hand, the rapid change of pressure in the irrigation line can be used to be able to detect the onset and end of an occlusion more quickly. Moreover, it is not necessary to evaluate two sensors with different time constants in a control device.

According to one embodiment of the invention, the irrigation line has an irrigation valve which, seen in the direction of flow, is arranged upstream of a handpiece for ophthalmosurgical treatment with a vibrating needle tip.

By suitable actuation of such an irrigation valve, it is possible to quickly terminate or resume the supply of an irrigation fluid. Moreover, the sensor can be arranged such that it can be acted upon by a pressure in the irrigation line, which pressure, seen in the direction of flow, is present upstream of the irrigation valve. This has the effect that it is possible to detect a fault condition of the ophthalmosurgical system in which the irrigation valve is closed and at the same time a suction pump is activated, which poses a danger to the eye. In this fault condition, the underpressure building up in the aspiration line is continued through the eye into the area of the irrigation line located between eye and irrigation valve. The entire eye is thus exposed to a dangerous underpressure. However, in the area of the irrigation line located upstream of the irrigation valve, as seen in the direction of flow, a normally high hydrostatic pressure is still present depending on the position of the irrigation fluid container, such that the sensor for detecting the differential pressure between irrigation line and aspiration line can detect a marked difference from the underpressure present in the aspiration line.

The device preferably has a venting valve in a venting line, which connects the irrigation line directly to the aspiration line. If, for example after an occlusion has been broken through, the vacuum pressure in the aspiration line increases again in the direction of the normal suction pressure, the venting line can be suitably opened by means of the venting valve, such that a rapid pressure compensation is possible and a drop in the suction pressure to too high a value is avoided.

According to another embodiment of the invention, the ophthalmosurgical measuring device has a control unit, wherein the sensor generates a signal that is associated with the differential pressure and that can be delivered to the control unit, which is able to control the flow of fluid in the irrigation line and/or aspiration line and/or an ultrasound energy for the handpiece. For example, the control unit, at the onset of an occlusion on the handpiece, can increase the ultrasound energy delivered for operating the handpiece and, at the end of the occlusion, can reduce the delivered ultrasound energy. By means of the increased ultrasound energy, a particle causing the blockage can be set particularly intensively into vibration, such that there is an increased probability of this particle being broken up. When this has finally been achieved, the ultrasound energy can be reduced after the end of the occlusion, in order to minimize the danger of damage to the capsular bag.

The sensor of the device according to the invention preferably has a bidirectionally movable element, such as a membrane, a spring tongue or a bar, whose position can be changed as a function of the differential pressure between the irrigation line and aspiration line, or whose force exerted on a force sensor can be detected as a function of the differential pressure between the irrigation line and aspiration line. Such a sensor with a high degree of sensitivity and a short response time can be produced. The sensor preferably has a time constant of T ≥ 10 ms at a pressure resolution of less than 666 kg/(ms²) (5 mmHg). To allow a noninvasive measurement, the measuring device can be designed such that the bend position of the bidirectionally movable element can be detected by a contactless displacement sensor.

The invention further relates to an ophthalmosurgical system with an ophthalmosurgical device as described above, an irrigation fluid container, a handpiece, and a suction pump for aspiration of aspiration fluid.

The invention further relates to a method for operating an ophthalmosurgical device as described above, wherein the differential pressure between irrigation line and aspiration line is detected by the sensor. The time gradient of the differential pressure profile is preferably determined. In this way, the change in pressure at the onset and at the end of an occlusion can be detected even more quickly. According to one embodiment of the invention, the signal of the gradient of the differential pressure profile is used to control the flow of fluid and/or the suction pump and/or the ultrasound energy delivered to the handpiece.

Further advantages and features of the invention are explained with reference to the attached drawings, in which:
- Figure 1: shows a schematic representation of an ophthalmosurgical system with an ophthalmosurgical measuring device not according to the invention;
- Figure 2: shows a schematic representation of the pressure profiles in an aspiration line and irrigation line of the system, and of the pressure profiles in a suction pump and an irrigation valve;
- Figure 3: shows a schematic representation of the differential pressure as a function of time at the pressure profiles shown in Figure 2;
- Figure 4A: shows a schematic representation of the control-technology relationships of a measuring device with two individual pressure sensors;
- Fig. 4B: shows a schematic representation of the control-technology relationships in the measuring device according to the invention;
- Figure 5: shows a schematic representation of the pressure profiles as a function of time when an occlusion is broken through; and
- Figure 6: shows a schematic representation of the profile of the differential pressure as a function of time during use of the measuring device according to the invention.

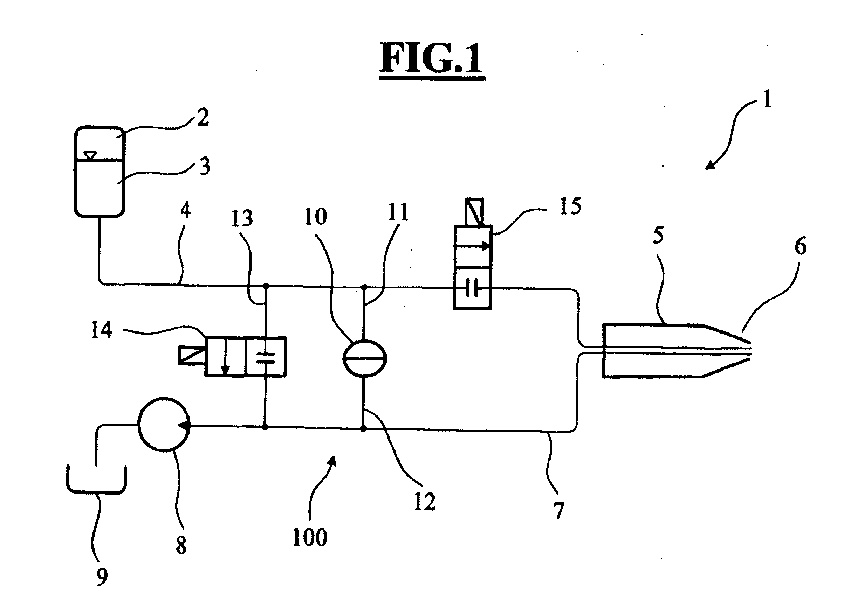

Figure 1 is a schematic representation of an ophthalmosurgical system 1 not itself according to the invention. An irrigation fluid container 2 contains an irrigation fluid 3 that can flow through an irrigation line 4 to a handpiece 5 with a tip 6. The tip 6 is designed in such a way that it is able, with the aid of a needle set in vibration by ultrasound, to break up a clouded and relatively hard lens of an eye into small fragments. The fluid located in the anterior chamber of the eye and the fragmented particles are guided through an aspiration line 7 to a suction pump 8, which discharges the fluid and the particles into a container 9. A sensor 10 is arranged between the irrigation line 4 and the aspiration line 7. In this device, the sensor is connected by way of a first line 11 to the irrigation line 4, and by way of a second line 12 to the aspiration line 7. It is thus possible for the sensor to detect a differential pressure between the irrigation line 4 and the aspiration line 7. However, according to the invention the sensor is designed in such a way that a first line 11 and a second line 12 are not present, such that the sensor 10 is connected directly to the irrigation line 4 and the aspiration line 7. At least part of the irrigation line 4, the differential pressure sensor 10 and at least part of the aspiration line 7 together form an ophthalmosurgical measuring device 100. A venting line 13, which is provided with a venting valve 14, can be connected in parallel with this measuring device 100. If the differential pressure measurement by means of the sensor 10 indicates that the occlusion has been broken through, the venting valve 14 can be activated in such a way that irrigation fluid 3 from the irrigation line 4 can pass through the venting line 13 into the aspiration line 7, in order to quickly lower the underpressure in the aspiration line 7.

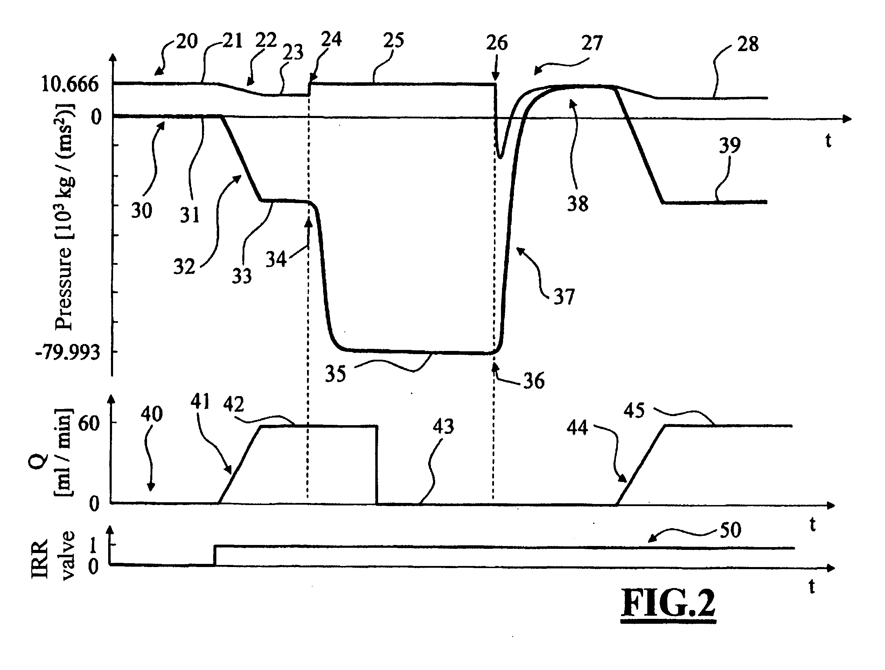

Figure 2 shows, in the upper area, the pressure profiles in an aspiration line and an irrigation line as a function of time. The upper curve 20 describes the profile of the irrigation pressure, while the curve 30 below this represents the profile of the aspiration pressure. It is assumed that, prior to the operation of a suction pump, the hydrostatic pressure in the irrigation line is ca. 10.666 kPa (= 80 mmHg), and the aspiration pressure is 0 kPa (= 0 mmHg), in connection with which it will be noted that these figures, and the figures given hereinbelow, serve only as examples, and higher or lower values are also possible. With the start-up of a suction pump (see reference sign 41 in Figure 1), the suction pressure in the aspiration line rises (see reference sign 32) to a stationary value 33. At the same time, the pressure in the irrigation line decreases (see reference sign 22) and likewise reaches a stationary value 23. At these stationary values 23 and 33, the suction pump works, for example, with a delivery volume of 60 milliliters per minute (see reference sign 42). If an occlusion occurs (see reference signs 24 and 34), the pressures in the irrigation line and aspiration line change. In the irrigation line, the pressure rises quickly again to the hydrostatic pressure (see reference sign 25), while the underpressure in the aspiration line rises relatively slowly, until it has reached a maximum level of, for example, -79.993 kPa (= -600 mmHg) (see reference sign 35). The suction pump can then be switched off (see reference sign 43). If the occlusion is broken through (see reference signs 26 and 36), the pressure in the irrigation line and aspiration line changes. In the irrigation line, there is a very rapid drop in pressure, shortly after which the pressure rises rapidly again and assumes the hydrostatic pressure (see reference sign 27). The underpressure in the aspiration line drops relatively quickly from the very high level of -79.993 kPa (= -600 mmHg) (see reference sign 37) and reaches the hydrostatic pressure (see reference sign 38). When the suction pump is returned to its previous suction capacity (see reference signs 44 and 45), the pressures in the irrigation line and aspiration line fall again to the levels prior to the occlusion (see reference signs 28 and 39). Throughout the cycle, an irrigation valve was at all times open in the irrigation line, such that the irrigation fluid was permanently available (see reference sign 50 in Figure 2).

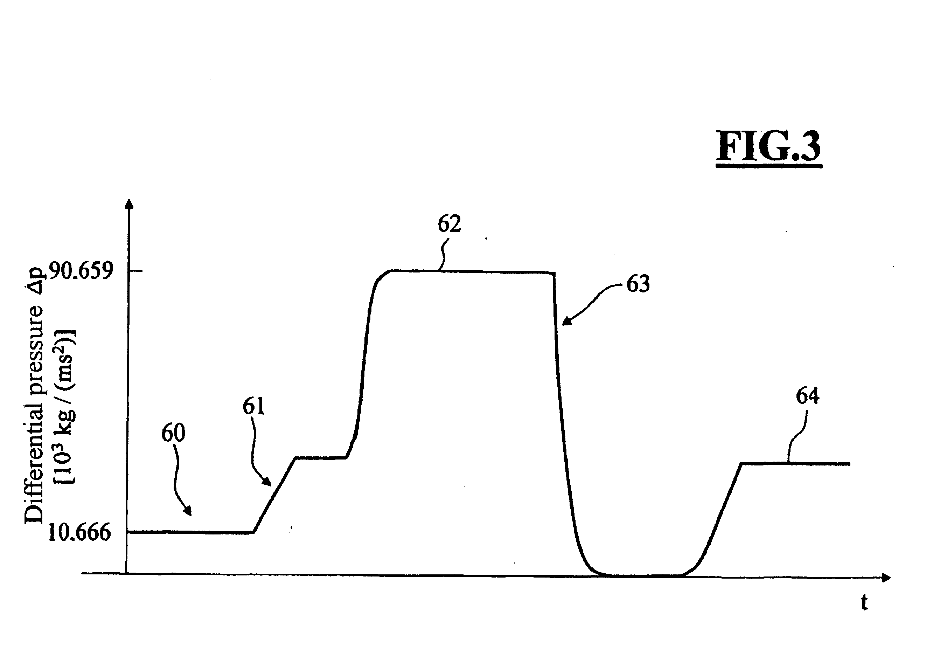

Figure 3 shows a profile 60 of the differential pressure between the irrigation line and the aspiration line analogously to the situation shown in Figure 2. Before the suction pump is switched on, the differential pressure has a relatively low value. With the suction pump switched on (see reference sign 61), the differential pressure between the irrigation line and the aspiration line rises. If the needle at the tip 6 of the handpiece 5 is blocked, the differential pressure rises quickly to a high value (see reference sign 62). When a particle is broken through and an occlusion ended (see reference signs 26 and 36 in Figure 2), the differential pressure drops very quickly (see reference sign 63 in Figure 3). After the suction pump is switched on, the differential pressure again reaches the level that existed prior to the occlusion (see reference sign 64).

The profile of the differential pressure in Figure 3 makes clear that the respective pressure in the irrigation line and aspiration line is not known. Neither a pressure in the aspiration line nor a pressure in the irrigation line is separately measured. Thus, no component of the ophthalmosurgical system is controlled on the basis of a separately measured irrigation pressure or aspiration pressure. The only available signal for controlling a system component originates from the differential pressure sensor. It is not possible to tell from the curve how this pressure profile determined by the differential pressure sensor is composed of the irrigation pressure and aspiration pressure. A reference to atmospheric pressure is not known. To evaluate this profile, it is possible to use either the directly measured differential pressure (see curve 60 in Figure 3) or a time gradient of the curve profile. The start and end of an occlusion in the needle of a handpiece can be identified very clearly and unambiguously from the curve 60. Such a profile can be used such that the needle of the handpiece can be set in vibration only when the differential pressure does not exceed a predetermined amount.

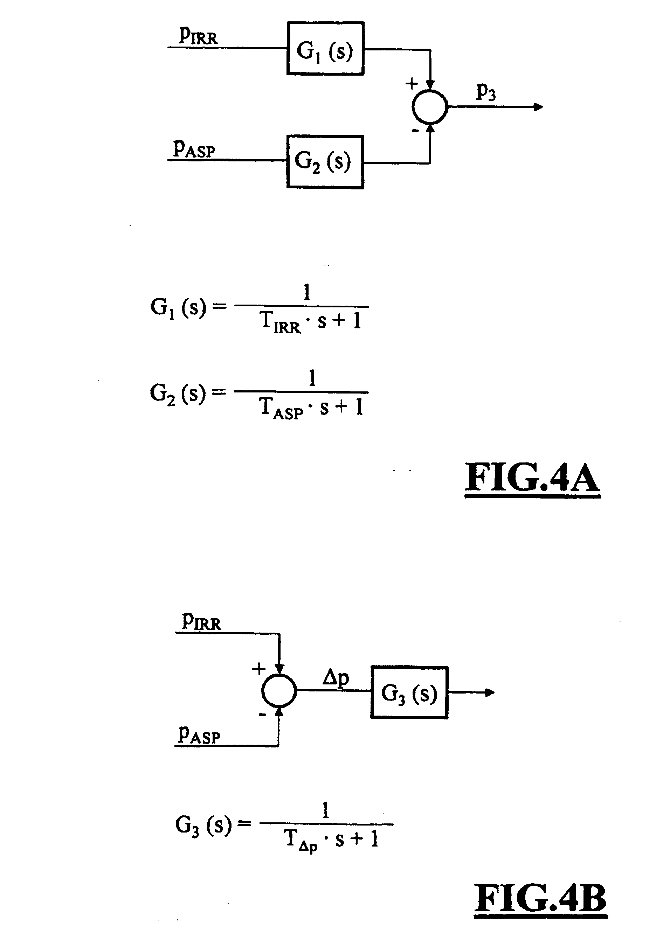

Fig. 4A is a schematic representation of the control technology relationship for a measuring device in which a pressure in the irrigation line and aspiration line is recorded separately. Fig. 4B is a schematic representation of the control technology relationship for the measuring device according to the invention in which a differential pressure between irrigation line and aspiration line is recorded. As can be seen from Figure 4A, a first-order delay element with the time constant T_{IRR} is assumed for the transfer function G₁(s) of the sensor for measuring the pressure in the irrigation line. A first-order delay element with the time constant T_{ASP} is assumed for the transfer function G₂(s) for the sensor of the pressure in the aspiration line. The processing of both signals yields a pressure p3. In the transfer functions, "s" stands for the complex variable. In the diagram shown in Figure 4B, the pressure in the irrigation line and the pressure in the aspiration line are fed to a differential pressure sensor, which determines from these a differential pressure Δp. The transfer function of this differential pressure sensor is assumed by a first-order delay element with the time constant T_{Δp}. The behavior in the differential pressure measurement can thus be detected with only one transfer function and one time constant.

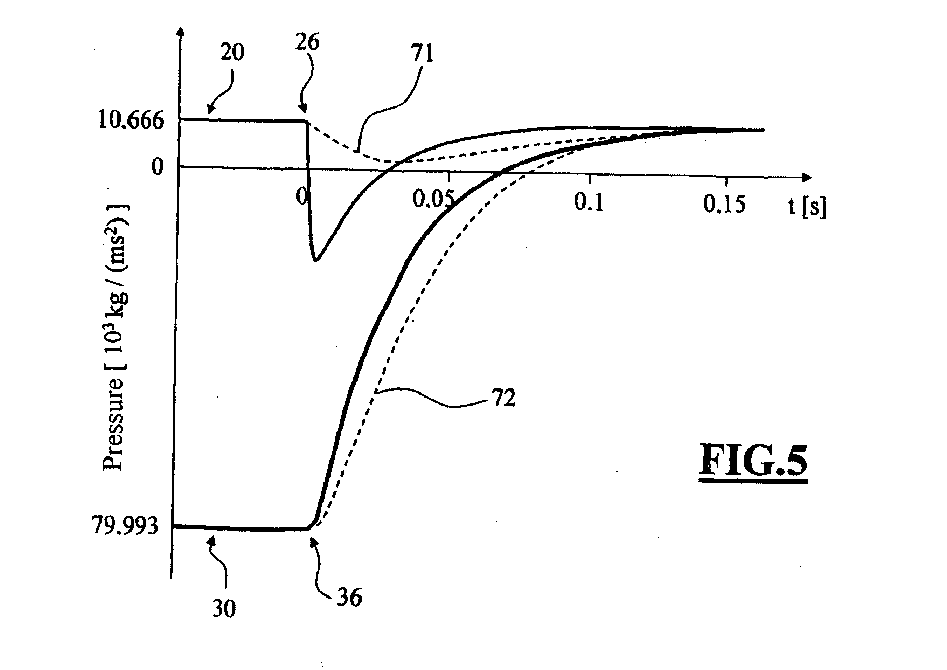

Figure 5 shows the time profile of the pressure curve 20 for the irrigation line and of the pressure curve 30 for the aspiration line. This is a small segment of the situation shown in Figure 2 at the end of an occlusion (see reference sign 26 for the pressure in the irrigation line and reference sign 36 for the pressure in the aspiration line). If the pressure profiles are detected separately with individual pressure sensors, i.e. with a pressure sensor for the irrigation line and a pressure sensor for the aspiration line, a pressure profile can be measured that is shown in each case by a broken line in Fig. 5. The broken line 71 shows the pressure profile for the irrigation line, while the broken line 72 shows the pressure profile for the aspiration line.

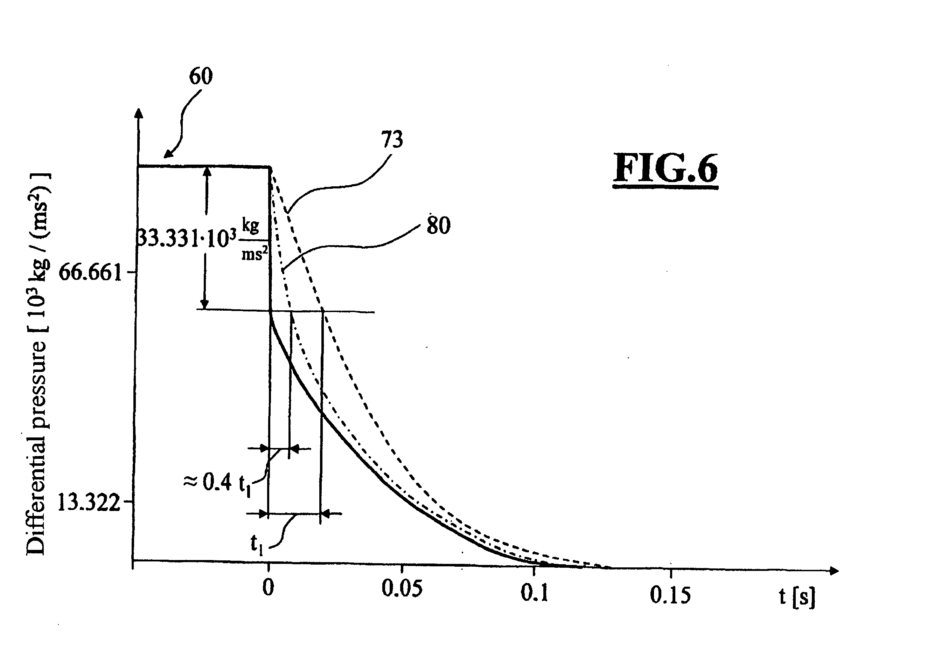

The pressure sensor that records the pressure in the irrigation line has a relatively large time constant at the level of T_{IRR} = 50 ms. This is due to the fact that, in the prior art, if a pressure sensor is indeed present at all in the irrigation line, it is used to detect the hydrostatic pressure of the irrigation fluid, such that the vertical position of the irrigation fluid container can be changed, if so required. Such a pressure sensor is relatively slow. It can be inferred from the profile of the curve 71 that the sharp drop in the irrigation pressure when the occlusion is broken through is barely detected, on account of the long time constant of the irrigation pressure sensor, with the result that the curve is "smudged". By contrast, the pressure sensor for the aspiration line is intended to be able to detect the pressure fluctuations relatively quickly, with the result that in most cases such a pressure sensor has a time constant with a relatively low value in the region of, for example, T_{ASP} = 10 ms. The profile of the curve 72 shows that the pressure profile in the aspiration line can be readily detected and, as a result, there not so much "smudging". Addition of the pressure values shown by the curves 71 and 72 according to the signal processing shown in Fig. 4A results in the sum profile shown by reference sign 73 (see Fig. 6). If the irrigation pressure sensor is omitted, and only the relatively quickly responding aspiration pressure sensor is used for the evaluation, a break-through of an occlusion cannot be detected quickly enough, because the aspiration pressure changes only relatively slowly.

If the differential pressure sensor according to the invention is used with signal processing according to Fig. 4B, wherein the sensor has a time constant of T_{ΔP} = 10 ms, this results in the curve profile indicated by reference sign 80 (see Figure 6). A comparison between the curves 73 and 80 shows that, with the curve 80, a change in the differential pressure of 33.331 kPa (= 250 mmHg) can be detected after only about 40% of the time that is needed when using two conventional pressure sensors. The measurement with the measuring device according to the invention is therefore much quicker, such that control of the flow characteristics and/or of the ultrasound energy for the handpiece can be initiated more quickly. The very rapid change in the irrigation pressure can be better detected by the differential pressure sensor, with the overall result that a more rapid reaction is possible to an occlusion being broken through.

## Claims

1. Ophthalmosurgical device (100) having:
- an irrigation line (4) through which irrigation fluid (3) can be transported,
- an aspiration line (7) through which aspiration fluid can be transported to a suction pump (8), and
- a sensor (10) with which a differential pressure between irrigation line (4) and aspiration line (7) can be detected, **characterised in that**
the sensor (10) is directly connected to the irrigation line (4) and the aspiration line (7).

2. Device (100) according to Claim 1, wherein the irrigation line (4) has an irrigation valve (15) which, seen in the direction of flow, is arranged upstream of a handpiece (5) for ophthalmosurgical treatment.

3. Device (100) according to Claim 2, wherein the sensor (10) is arranged such that it can be acted upon by a pressure in the irrigation line (4), which pressure, seen in the direction of flow, is present upstream of the irrigation valve (15).

4. Device (100) according to one of Claims 1 to 3, wherein the device (100) has a venting valve (14) in a venting line (13), which connects the irrigation line (4) directly to the aspiration line (7).

5. Device (100) according to one of Claims 1 to 4, which device (100) has a control unit, wherein the sensor (10) generates a signal that is associated with the differential pressure and that can be delivered to the control unit, which is able to control the flow of fluid in the irrigation line (4) and/or aspiration line (7) and/or an ultrasound energy for the handpiece (5).

6. Device (100) according to Claim 5, wherein the control unit, at the onset of an occlusion on the handpiece (5), increases the ultrasound energy delivered for operating the handpiece (5) and, at the end of the occlusion, reduces the delivered ultrasound energy.

7. Device (100) according to one of Claims 1 to 6, wherein the sensor (10) has a bidirectionally movable element, such as a membrane, a spring tongue or a bar, whose position can be changed as a function of the differential pressure between the irrigation line and aspiration line, or whose force exerted on a force sensor can be detected as a function of the differential pressure between the irrigation line (4) and aspiration line (7).

8. Device (100) according to one of Claims 1 to 7, wherein the sensor (10) has a time constant T ≥ 10 ms at a pressure resolution of less than .666 kPa

9. Device (100) according to either of Claims 7 and 8, wherein the bend position of the element can be detected by a contactless displacement sensor.

10. Ophthalmosurgical system (1) with a measuring device (100) according to one of Claims 1 to 9, an irrigation fluid container (2), a handpiece (5) for ophthalmological treatment, and a suction pump (8) for aspiration of fluid.

## Patentansprüche

1. Ophthalmochirurgische Vorrichtung (100), aufweisend:
- eine Irrigationsleitung (4), durch welche Irrigationsfluid (3) transportierbar ist,
- eine Aspirationsleitung (7), durch welche Aspirationsfluid zu einer Saugpumpe (8) transportierbar ist, und
- einen Sensor (10), mit dem ein Differenzdruck zwischen der Irrigationsleitung (4) und Aspirationsleitung (7) erfassbar ist, **dadurch gekennzeichnet, dass**
der Sensor (10) direkt mit der Irrigationsleitung (4) und der Aspirationsleitung (7) verbunden ist.

2. Vorrichtung (100) nach Anspruch 1, wobei die Irrigationsleitung (4) ein Irrigationsventil (15) aufweist, welches in Strömungsrichtung gesehen vor einem Handstück (5) für eine ophthalmochirurgische Behandlung angeordnet ist.

3. Vorrichtung (100) nach Anspruch 2, wobei der Sensor (10) so angeordnet ist, dass auf ihn ein Druck in der Irrigationsleitung (4) wirken kann, welcher in Strömungsrichtung gesehen vor dem Irrigationsventil (15) besteht.

4. Vorrichtung (100) nach einem der Ansprüche 1 bis 3, wobei die Vorrichtung (100) ein Belüftungsventil (14) in einer Belüftungsleitung (13) aufweist, welche die Irrigationsleitung (4) direkt mit der Aspirationsleitung (7) verbindet.

5. Vorrichtung (100) nach einem der Ansprüche 1 bis 4, welche eine Steuereinheit aufweist, wobei der Sensor (10) ein dem Differenzdruck zugehöriges Signal erzeugt, welches der Steuereinheit zuführbar ist, mit welcher die Fluidströmung in der Irrigationsleitung (4) und/oder Aspirationsleitung (7) und/oder eine Ultraschallenergie für das Handstück (5) steuerbar ist.

6. Vorrichtung (100) nach Anspruch 5, wobei die Steuereinheit bei Beginn einer Okklusion am Handstück (5) die zugeführte Ultraschallenergie für das Betreiben des Handstückes (5) erhöht und bei Ende der Okklusion die zugeführte Ultraschallenergie vermindert.

7. Vorrichtung (100) nach einem der Ansprüche 1 bis 6, wobei der Sensor (10) ein bidirektional bewegbares Element wie eine Membrane, eine Federzunge oder einen Balken aufweist, dessen Position in Abhängigkeit vom Differenzdruck zwischen der Irrigationsleitung und Aspirationsleitung verlagerbar oder dessen auf einen Kraftsensor ausgeübte Kraft in Abhängigkeit vom Differenzdruck zwischen der Irrigationsleitung (4) und Aspirationsleitung (7) erfassbar ist.

8. Vorrichtung (100) nach einem der Ansprüche 1 bis 7, wobei der Sensor (10) eine Zeitkonstante T ≥ 10 ms bei einer Druckauflösung von weniger als 0,666 Pa aufweist.

9. Vorrichtung (100) nach einem der Ansprüche 7 und 8, wobei die Biegeposition des Elementes durch einen berührungslosen Wegsensor erfassbar ist.

10. Ophthalmochirurgisches System (1) mit einer Messvorrichtung (100) nach einem der Ansprüche 1 bis 9, einem Irrigationsfluidbehälter (2), einem Handstück (5) für eine ophthalmologische Behandlung und einer Saugpumpe (8) zum Absaugen von Fluid.

## Revendications

1. Dispositif ophtalmo-chirurgical (100) comprenant :
- une conduite (4) d'irrigation à travers laquelle du fluide (3) d'irrigation peut être transporté,
- une conduite (7) d'aspiration à travers laquelle du fluide d'aspiration peut être transporté jusqu'à une pompe (8) d'aspiration, et
- un capteur (10) au moyen duquel une pression différentielle entre conduite (4) d'irrigation et conduite (7) d'aspiration peut être détecté,
**caractérisé en ce que** le capteur (10) est directement relié à la conduite (4) d'irrigation et à la conduite (7) d'aspiration.

2. Dispositif (100) selon la revendication 1, la conduite (4) d'irrigation étant dotée d'une vanne (15) d'irrigation qui, vue dans la direction de l'écoulement, est disposée en amont d'une pièce (5) à main servant au traitement ophtalmo-chirurgical.

3. Dispositif (100) selon la revendication 2, le capteur (10) étant disposé de telle façon qu'il peut être soumis à l'action d'une pression dans la conduite (4) d'irrigation, ladite pression, vue dans la direction de l'écoulement, étant présente en amont de la vanne (15) d'irrigation.

4. Dispositif (100) selon l'une des revendications 1 à 3, le dispositif (100) étant doté d'une soupape (14) de purge sur une conduite (13) de purge, qui relie directement la conduite (4) d'irrigation à la conduite (7) d'aspiration.

5. Dispositif (100) selon l'une des revendications 1 à 4, ledit dispositif (100) étant doté d'une unité de commande, le capteur (10) générant un signal qui est associé à la pression différentielle et qui peut être transmis à l'unité de commande, qui est capable de réguler le débit de fluide dans la conduite (4) d'irrigation et/ou la conduite (7) d'aspiration et/ou une énergie ultrasonore pour la pièce (5) à main.

6. Dispositif (100) selon la revendication 5, l'unité de commande, au début d'une occlusion sur la pièce (5) à main, augmentant l'énergie ultrasonore transmise pour faire fonctionner la pièce (5) à main et, à la fin de l'occlusion, réduisant l'énergie ultrasonore transmise.

7. Dispositif (100) selon l'une des revendications 1 à 6, le capteur (10) comprenant un élément mobile dans deux directions, comme une membrane, un lame de ressort ou une barre, dont la position peut être modifiée en fonction de la pression différentielle entre la conduite d'irrigation et la conduite d'aspiration, ou dont la force exercée sur un capteur de force peut être détectée en fonction de la pression différentielle entre la conduite (4) d'irrigation et la conduite (7) d'aspiration.

8. Dispositif (100) selon l'une des revendications 1 à 7, le capteur (10) présentant une constante de temps T ≥ 10 ms à une résolution en pression inférieure à 0,666 kPa.

9. Dispositif (100) selon l'une quelconque des revendications 7 et 8, la position du coude de l'élément pouvant être détectée par un capteur de déplacement sans contact.

10. Système ophtalmo-chirurgical (1) doté d'un dispositif (100) de mesure selon l'une des revendications 1 à 9, d'un récipient (2) de fluide d'irrigation, d'une pièce (5) à main pour traitement ophtalmo-chirurgical et d'une pompe (8) d'aspiration servant à l'aspiration de fluide.
